# EUROPEAN PATENT APPLICATION

(11) **EP 2 853 596 A1**
(43) Date of publication of application: **01.04.2015**
(21) Application number: 13186746.7
(22) Date of filing: 30.09.2013
(51) Int. Cl.: C12N 15/113, A61K 31/713, A61P 35/00

(54) **Protein phosphatase inhibitor**

(71) Applicant: IKBT (Institut für Klinische Biomedizinische Forschung Thurgau), 8501 Frauenfeld (CH)
(72) Inventor: Makia, Ntoh Divine Oscar, 8512 Thundorf (CH)
(74) Representative: Van Breda, Jacobus

(57) **Abstract**

The invention pertains to a combination of a PPEF inhibitor capable of causing apoptosis in cancer cells and an active pharmaceutical ingredient capable of causing apoptosis in cancer cells.

## Description

The present invention relates to a pharmaceutical agent for treating cancer cells, combinations thereof, and its use as a medicament.
Some kinases and phosphatases have been reported to play a role in cell survival as is disclosed by MacKeigan et al (in Nature Cell Biology, Vol. 7, Nr. 6, June 2005, pp. 591-604). Examples of phosphatases include the serine/threonine protein phosphatase such as PP1, PP2A, PP4 and PP6, and also a protein phosphatase having an EF hand like PPEF2. Andreeva et al (in "Cell Mol Life Sci (2009), 66, pp. 3103-3110") and Hu et al (in "Mol.Cancer Ther., 2009, 8, pp. 3024-3035) indicate the positive responses to cell survival by PPEF1 and PPEF2.

WO 02/076989 discloses cantharidin analogues capable of inhibiting protein phosphatase.

The objective of the present invention is to provide a novel treatment, thereby using a target different from the prior art.

The present invention pertains to a combination of a PPEF inhibitor capable of causing apoptosis in cancer cells and an active pharmaceutical ingredient capable of causing apoptosis in cancer cells. The combination of the invention provides considerable apoptosis of cancer cells. This combined treatment generally reduces the viability of cancer cells more than the active pharmaceutical ingredient alone. Generally, the combination of the invention provides for a synergistic viability reduction in cancer cells, i.e. the overall viability reduction caused by the inventive combination is higher than the sum of the viability reduction caused by each of the components of that combination. Moreover, the PPEF inhibitor per se may cause apoptosis in cancer cells. By using the PPEF inhibitor in the combination of the invention the amount of active pharmaceutical ingredient may be reduced while maintaining or even improving the apoptosis level. In addition, the PPEF inhibitor is capable of sensitizing cancer cells not being receptive to the active pharmaceutical ingredient alone. This sensitization allows the active pharmaceutical ingredient to cause apoptosis in cancer cells.

In the context of the present application the term "combination" refers to a composition comprising both the PPEF inhibitor and the active pharmaceutical ingredient, or to a plurality of pharmaceutical compositions comprising both the inhibitor and the pharmaceutical ingredient in two or more different compositions. The present invention therefore also pertains to a kit-of-parts comprising a PPEF inhibitor capable of causing apoptosis in cancer cells and an active pharmaceutical ingredient capable of causing apoptosis in cancer cells. The plurality of compositions of the invention may be administered to a patient simultaneously and/or consecutively.

The PPEF inhibitor can be any compound capable of inhibiting the expression of a protein phosphatase having an EF hand (PPEF) in the cancer cell. With "inhibitor" it is meant to refer to compounds capable of reducing or diminishing the concentration or activity of all PPEFs or of a particular PPEF in the cells. The PPEF in accordance with the invention may be any PPEF known in the art. Examples of such PPEFs include PPEF1 and PPEF2 as well as variants of these PPEFs like the PPEF1 variants described in the EP 1 903 109. The PPEF inhibitor of the invention can be any compound capable of inhibiting a PPEF in a cell. The PPEF inhibitor may preferably inhibit PPEF1 and/or PPEF2, more preferably PPEF1. The gene sequence of human PPEF1 is expressed in SEQ ID NO 3, and its corresponding RNA in SEQ ID NO 4. The PPEF inhibitor of the invention is capable to inhibit, interfere or bind to the nucleotide sequences SEQ ID NO 3 and/or 4. In one embodiment of the invention, the PPEF inhibitor may be an oligonucleotide, in particular an oligonucleotide selected from an antisense oligonucleotide (AONT), a small interfering RNA oligonucleotide (SiRNA), and a triplex forming oligonucleotide (TFO). The different oligonucleotides are effective inhibitors of PPEF expression in the cell through differing pathways. The antisense oligonucleotide is capable of binding to the pre-mRNA of PPEF, therewith preventing translation to PPEF. The SiRNA oligonucleotide influences the RNA interference (RNAi) pathway, where it interferes with the expression of the PPEF gene. The triplex forming oligonucleotide binds to a groove of duplex RNA forming a triplex DNA structure, which is reported to inhibit DNA transcription and replication, generate site-specific mutations, cleave DNA and/or induce homologous recombination. In another embodiment, the PPEF inhibitor is a small chemical compound capable of interfering or modulating the expression of a PPEF protein, in particular the PPEF1 protein. In a preferred embodiment the PPEF inhibitor is a SiRNA oligonucleotide. In a more preferred embodiment the PPEF inhibitor is a SiRNA oligonucleotide selected from SEQ ID NO 1 and SEQ ID NO 2.

The invention further pertains to a PPEF inhibitor being capable of reducing the viability of cancer cells by at least 10% compared to a treatment without the PPEF inhibitor. The viability can be determined by methods described elsewhere in this description. The PPEF inhibitor of the invention is preferably an oligonucleotide selected from an antisense oligonucleotide (AONT), a small interfering RNA oligonucleotide (SiRNA), and a triplex forming oligonucleotide (TFO), preferably a small interfering RNA oligonucleotide. In a more preferred embodiment the PPEF inhibitor is a SiRNA oligonucleotide selected from SEQ ID NO 1 and SEQ ID NO 2.

The active pharmaceutical ingredient of the invention may be any compound capable of causing apoptosis in cancer cells known in the art. Examples of such active pharmaceutical ingredients include alkylating agents, anti-metabolites, anti-microtubule agents, topoisomerase inhibitors, antibody drug conjugates, monoclonal antibodies and tyrosine-kinase inhibitors (TKI). Examples of alkylating agents include nitrogen mustards like mechlorethamine, cyclophosphamide, melphalan, chlorambucil, ifosfamide and busulfan; nitrosoureas such as N-Nitroso-N-methylurea (MNU), carmustine (BCNU), lomustine (CCNU) and semustine (MeCCNU), fotemustine and streptozotocin; tetrazines like dacarbazine, mitozolomide and temozolomide; aziridines like thiotepa, mytomycin and diaziquone (AZQ); non-classical alkylating agents such as procarbazine and hexamethylmelamine; and cisplatins and derivatives or platinum-based pharmaceutical ingredients. Preferred alkylating agents are platinum-based pharmaceutical ingredients such as cisplatin, carboplatin and oxaliplatin. Examples of anti-metabolites include anti-folates such as methotrexate and pemetrexed; fluoropyrimidines like fluorouracil and capecitabine; deoxynucleoside analogues such as cytarabine, gemcitabine, decitabine, Vidaza, fludarabine, nelarabine, cladribine, clofarabine and pentostatin; and thiopurines like thioguanine and mercaptopurine. Examples of anti-microtubule agents include Vinca alkaloids and taxanes. Examples of topoisomerase inhibitors include topisomerase type I inhibitors like irinotecin, camptothecin (CPT), topotecan, indotecan, and indimitecan; and topisomerase type II inhibitors such as amsacrine, etoposide, etoposide phosphate, teniposide and doxorubicin. Examples of antibody drug conjugates include brentuximab vedotin and trastuzumab emtansine. Examples of monoclonal antibodies include bevacizumab, cetaximab, panitumumab, trastuzumab, rituximab, alemtuzumab and gemtuzumab. Examples of tyrosine-kinase inhibitor include imatinib, gefitinib, erlotinib and sunitinib.

In the combination of the invention, the alkylating agents and the topoisomerase inhibitors are preferred. More preferably, the active pharmaceutical ingredient is selected from platinum-based pharmaceutical ingredients and topoisomerase inhibitors. More preferably the active pharmaceutical ingredient is selected from cisplatin, comptothecin and etoposide.

In another aspect of the invention, the IC50 value of the combination is at least 5 times lower than the IC50 value of the active pharmaceutical ingredient alone. In a preferred embodiment of the invention, the IC50 value of the combination is at least 10 times lower than the IC50 value of the active pharmaceutical ingredient alone. More preferably, the IC50 value of the combination is at least 12 times lower, and most preferably at least 15 times lower than the IC50 value of the active pharmaceutical ingredient alone. The IC50 value is a term generally known by the skilled person in the art. In the context of this application, the IC50 value refers to the concentration at which the viability of the cancer cells has decreased to 50%. This value can be determined with various techniques. Examples of such techniques include the so-called PrestoBlue® cell viability assay protocol as is provided by Life Technologies, and the MTT cell viability assay protocol as is provided by Biotium. The preferred method for determining the IC50 value is the PrestoBlue® cell viability assay protocol.

In another aspect of the invention, the combination of the invention causes a viability reduction in cancer cells, which is higher than the viability reduction by the pharmaceutical ingredient alone. Preferably, the viability reduction in cancer cells of the combination of the invention is at least 10% higher, more preferably at least 20 % higher, and most preferably at least 25% higher than the viability reduction by the pharmaceutical ingredient alone. The reduction in viability of cancer cells can be determined with any suitable method known in the art. A preferred method is the so-called ApoTox-Glo^{™} Triplex Assay, which is a standard test provided by Promega.

Additionally or alternatively, the combination of the invention causes an apoptosis level in cancer cells, which is higher than the apoptosis level by the pharmaceutical ingredient alone. Preferably, the apoptosis level in cancer cells of the combination of the invention is at least 10% higher, more preferably at least 20 % higher, and most preferably at least 25% higher than the apoptosis level by the pharmaceutical ingredient alone. The increase in apoptosis level of cancer cells can be determined with any suitable method known in the art. A preferred method is the so-called ApoTox-Glo^{™} Triplex Assay, and in particular the caspase activation test, which is a standard test provided by Promega.

In one embodiment of the invention, the molar ratio between the PPEF inhibitor and the active pharmaceutical ingredient is at most 10:1, preferably at most 5.1, and most preferably at most 2:1, and generally at least 1: 20, preferably at least 1:10, more preferably at least 1:5, and most preferably at least 1:2.

The invention further pertains to a pharmaceutical composition comprising the combination of the invention, and a pharmaceutically acceptable carrier. The term "pharmaceutical composition" or "pharmaceutical formulation" refers to a preparation which is in such form as to permit the biological activity of an active ingredient contained therein to be effective, and which contains no additional components which are unacceptably toxic to a subject to which the formulation would be administered. A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In another embodiment of the invention, the combination of the invention is divided over two or more pharmaceutical compositions, wherein the PPEF inhibitor of the invention is comprised in one pharmaceutical composition and the active pharmaceutical ingredient is comprised in a second pharmaceutical composition. In this way the PPEF inhibitor and the active pharmaceutical ingredient can be administered to a patient consecutively. It is also envisaged to provide compositions comprising part of the total amount of the PPEF inhibitor and/or the active pharmaceutical ingredient.

In one embodiment, the invention pertains to the use of the combination of the invention or the pharmaceutical composition of the invention as a medicament. In yet another embodiment, the invention pertains to the use of the combination of the invention or the pharmaceutical composition of the invention in the treatment of cancer.

The invention further pertains to a PPEF inhibitor for use as a medicament, the PPEF inhibitor being capable of reducing the viability of cancer cells by at least 10% compared to a treatment without the PPEF inhibitor. In a preferred embodiment, the invention pertains to a PPEF inhibitor for use in cancer treatment, the PPEF inhibitor being capable of reducing the viability of cancer cells by at least 10% compared to a treatment without the PPEF inhibitor. The inventors have shown that a PPEF inhibitor can be advantageously used as a medicament, in particular as a cancer treatment, e.g. by causing apoptosis to cancer cells per se, by exhibiting synergistic apoptosis when used in combination with a cancer drug, and it is capable of sensitizing cells that otherwise would not have been receptive to a conventional drug. Preferably, the PPEF inhibitor a SiRNA oligonucleotide. Particularly suitable SiRNA oligonucleotides are described above.

The invention further pertains to the use of a PPEF inhibitor in the treatment of cancer, the PPEF inhibitor being capable of reducing the viability of cancer cells by at least 10% compared to a treatment without the PPEF inhibitor. Preferably, the viability of cancer cells is reduced by at least 15%, more preferably at least 20%, and most preferably at least 25% compared to a treatment without the PPEF inhibitor. The viability can be determined using any method described above.

In a further embodiment, the invention pertains to the use of a PPEF inhibitor to sensitize proliferative cells. In the context of this application, the term "proliferative cells" refers to cells which grow and increase in number relatively rapidly. Examples of diseases in which proliferative cells play a key role include cancer, atherosclerosis, rheumatoid arthritis, psoriasis, idiophatic pulmonary fibrosis, scleroderma and cirrhosis to the liver. It is believed that the expression of PPEF1 protein plays an important role in the survival of these proliferative cells. The PPEF inhibitor of the present invention is generally capable of reducing the growth of such cells significantly and/or to sensitize such cells to a second treatment with conventional pharmaceutical ingredients. Moreover, the PPEF inhibitor of the invention is generally capable of sensitizing proliferative cells to pharmaceutical ingredients, to which the cells are resistant. The term "resistant" means that the pharmaceutical ingredient does not have a significant effect, e.g. reduction in growth or apoptosis, on the resistant proliferative cells. Preferably, the invention pertains to a PPEF inhibitor for use in the treatment of proliferative diseases, in particular for use in cancer, atherosclerosis, rheumatoid arthritis, psoriasis, idiophatic pulmonary fibrosis, scleroderma and cirrhosis to the liver. In another preferred embodiment, the invention pertains to a PPEF inhibitor for use in sensitizing resistant proliferative cells to an active pharmaceutical ingredient capable of causing apoptosis in non-resistant proliferative cells.

In a further embodiment, the invention pertains to the use of a PPEF inhibitor of the invention to sensitize proliferative cells for the treatment with an active pharmaceutical ingredient capable of causing apoptosis in proliferative cells. Preferably, the PPEF inhibitor is capable of reducing the viability of proliferative cells by at least 10% compared to a treatment without the PPEF inhibitor. Preferably, the viability of proliferative cells is reduced by at least 15%, more preferably at least 20%, and most preferably at least 25% compared to a treatment without the PPEF inhibitor.

In yet a further embodiment, the invention pertains to the use of a PPEF inhibitor to sensitize cancer cells which are resistant to an active pharmaceutical ingredient capable of causing apoptosis in non-resistant cancer cells. By treatment with the PPEF inhibitor of the invention the resistant cancer cells are generally sensitized such that a subsequent treatment with the active pharmaceutical ingredient causes apoptosis to the resistant cancer cells.

In a further embodiment, the invention pertains to the use of a PPEF inhibitor of the invention to sensitize cancer cells for the treatment with an active pharmaceutical ingredient capable of causing apoptosis in cancer cells. Preferably, the PPEF inhibitor is capable of reducing the viability of cancer cells by at least 10% compared to a treatment without the PPEF inhibitor. Preferably, the viability of cancer cells is reduced by at least 15%, more preferably at least 20%, and most preferably at least 25% compared to a treatment without the PPEF inhibitor. The viability can be determined using any method described above. By "sensitize" or "sensitizing" it is meant to describe the process of treating a cell, in particular a cancer cell, whereby the treatment causes the cell to be sensitive to an effective treatment with a pharmaceutical compound of which the compound on its own would not have an effect on the treated cell, or would cause a viability reduction in the cancer cell of less than 5%, preferably less than 2%, more preferably less than 1%, and most preferably no viability reduction.

The use of a PPEF inhibitor in the treatment of cancer can be accompanied (simultaneous exposure) or followed (consecutive exposure) by a radiation treatment. Generally, the radiation treatment causes apoptosis of the cancer cells. The combined treatment may provide for a more effective treatment of the cancer cells.

The term "cancer" as used herein refers to proliferative diseases, such as lymphomas, lymphocytic leukemias, lung cancer, non-small-cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

An "effective amount" of an agent, e.g., a pharmaceutical formulation, refers to an amount effective, at dosages and for periods of time necessary, to achieve the desired therapeutic or prophylactic result.

The invention further pertains to an expression vector comprising the oligonucleotide of the invention.

A further embodiment of the invention is a host cell comprising the oligonucleotide of the invention or the expression vector of the invention.

A yet further embodiment of the invention pertains to a method of producing a PPEF inhibitor, the PPEF inhibitor being an oligonucleotide comprising culturing the host cell under conditions suitable for the expression of said PPEF inhibitor and isolating said PPEF inhibitor.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

As used herein, the expressions "cell", "cell line", and "cell culture" are used interchangeably and all such designations include progeny. Thus, the words "transfectants" and "transfected cells" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

A brief description of the Figures is given below.
- **Figure 1:**: Titration of camptothecin on U20S cells.
- **Figure 2A-B:**: Evaluation of SiRNA set by viability test. A) Single SiRNA oligonucleotides were transfected in cells 24hrs post seeding and 24hrs post transfection were treated with or without 250nM CPT. B) Different combinations of the different oligonucleotides were prepared according to our protocol and the cells were transfected in triplicates. 24hrs post transfection, cells were treated with or without 250nM CPT. Wells not treated with CPT were provided the same amount of empty culture medium to ensure the same end dilution of the SiRNA.
- **Figure 2C:**: Combination therapy demonstrating left shift on U2OS cells treated with CPT.
- **Figure 2D:**: Combination therapy demonstrating left shift on U2OS cells treated with Etoposide.
- **Figure 3:**: Cytotoxicity assay. Unspecific SiRNA transfection control (Allstar), Allstar transfection followed by 250nM camptothecin (CPT), 250nM CPT treated cells, SiRNA oligo specific for target phosphatase PPEF1 with or without CPT.
- **Figure 4A-B:**: Apoptosis assay of target phosphatase SiRNA treated cells. (A) Apoptosis analysis 48hrs post transfection. (B) Apoptosis analysis 72hrs post transfection (Cell control) untreated U20S cells, (Allstar) unspecific SiRNA transfection control, (CPT) camptothecin 250nM, SiRNA specific for target (PPEF1) phosphatase.
- **Figure 5A:**: Western blot phosphorylation status of histone H2AX. A time course experiment was performed using U2OS cells in which equal number of cells was plated and transfected with the indicated SiRNAs. 24 hrs post transfection, cells treated with and without 250nM CPT and the cells were collected for protein isolation at indicated time points. After protein quantitation, equal amount (10ug) of total protein was probed with the antibodies indicated above. (GAPDH was used to demonstrate equal loading).
- **Figure 5B:**: Western blot phosphorylation status of histone H2AX. A time course experiment was performed using U20S cells in which equal number of cells was plated and transfected with the indicated SiRNAs. 24 hrs post transfection, cells treated with and without 250nM CPT and the cells were collected for protein isolation at indicated time points. After protein quantitation, equal amount (10ug) of total protein was probed with the antibodies indicated above. (GAPDH was used to demonstrate equal loading).
- **Figure 6A:**: Camptothecin (CPT) treatment of DU145 prostate cancer wild-type cell line. CPT was titrated on DU145 wild-type cells and 48hrs post CPT treatment, the cells were examined for the loss of viability compared to control (untreated)
- **Figure 6B:**: Camptothecin treatment of CPT approved resistant cells. CPT was titrated on DU145/RCO.1 cells reported to be resistant to CPT. 48hrs post CPT treatment, the cells were examined for the loss of viability compared to control (untreated)
- **Figure 6C:**: Camptothecin treatment of CPT approved resistant cells after PPEF1 inhibition. 24hrs post PPEF1 SiRNA treatment, CPT was titrated on DU145/RCO.1 cells reported to be resistant to CPT. 48hrs post CPT treatment, the cells were examined for the loss of viability compared to control (untreated)
The invention is exemplified in the following Examples.

### Examples

### Examples 1 and 2

### Materials

The oligonucleotides used in the experiments described below have been tabulated in Table 1. All oligonucleotides were obtained from Qiagen.

**Table 1**

| Example | Name | SEQ ID NO |
|---|---|---|
| 1 | PPEF1-2 | 1 |
| 2 | PPEF1-5 | 2 |
| A | PPEF1-1 | |
| B | PPEF1-6 | |

### Primary screening

The primary screening was designed to enable us to easily filter the SiRNA libraries (phosphatase and HDACs) and to focus on novel hits. Therefore, we established the PrestoBlue^{™} cell viability assay protocol to evaluate any effect caused by the SiRNA treatment. PrestoBlue^{™} reagent is a resazurin-based solution that functions as a cell viability indicator by using the reducing power of living cells to quantitatively measure the proliferation of cells. This reagent contains a cell-permeant compound that is blue in colour and virtually non-fluorescent. When added to cells, the PrestoBlue^{™} reagent is modified by the reducing environment of the viable cell, turns red in colour and becomes highly fluorescent. This change can be detected using fluorescence or absorbance measurements. Additionally, the simplicity of this assay is compatible to high throughput using a 96-well plate containing the cells and the compound(s) to be tested. The PrestoBlue^{™} reagent is added directly to cells and incubated at 37°C for up to 2 hours. The plate is then transferred to a fluorescence reader to measure the fluorescent signal. Results are evaluated by plotting signal versus compound concentration.

### Titration of CPT

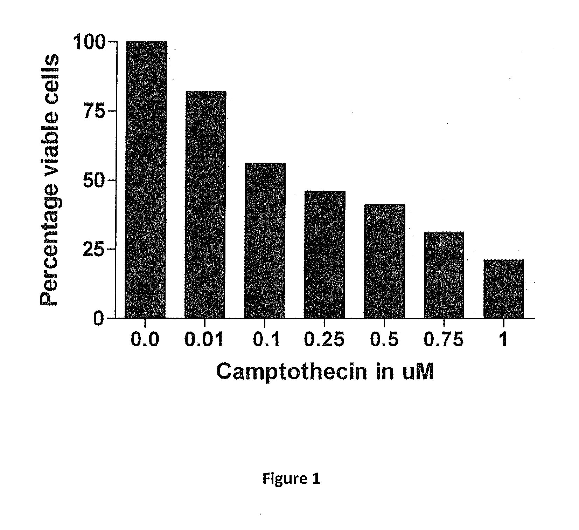

In order to determine the effect of a combination of SiRNA knockdown and the DNA damaging agent camptothecin (CPT), we performed a titration of the CPT on the target cells. Usually, 1µM of CPT with incubation between 1hr to 4hrs is used in some experimental set ups. Since we intended to evaluate the effect of SiRNA knockdown on the cells a lower concentration is demanded. The CPT was titrated on the U2OS cells and 48hrs post titration the effect on the viability of the cells was analyzed. The results are shown in Figure 1.

### Titration of SiRNA

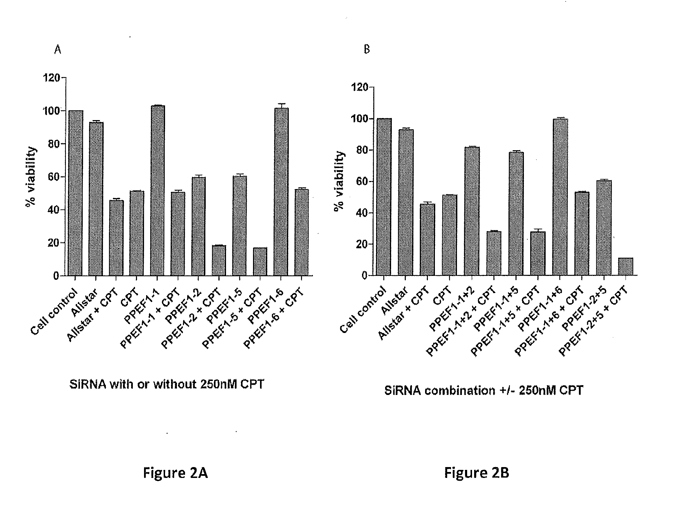

The SiRNA was initially titrated on the cells in order to decide the best concentration to be applied. The titration was performed with 5nM, 10nM, 15nM and 20nM of SiRNA and according to the preliminary data (data not shown) we decided to work with 5nM concentration. Additionally, we tested the SiRNA oligonucleotides on their own as well as different combinations of the SiRNA oligonucleotides targeting the same mRNA. For a reliable experiment at least 2 out of the 4 SiRNA oligonucleotides should lead to similar results. Figure 2a below depicts our assertion of reliability and Figure 2b indicates an example of the different combinations that were examined. We therefore selected only oligonucleotides that induced reliable reduction of viability to the cells in 3 separate experiments for further analysis. We observed that the oligonucleotides of Comparative Example A (PPEF1-1) and Comparative Example B (PPEF1-6) had no effect on cell viability and their combination with 250nM CPT only confirmed the effect of CPT mediated reduction in viability when compared to CPT treatment alone. In contrast the oligonucleotides of Examples 1 (PPEF1-2) and 2 (PPEF1-5) led to reduction in viability of about 40% each and when their treatment was followed by treatment with CPT, there was an additional reduction in viability amounting to a total of about 80%. Evaluating the combination of the different SiRNA oligonucleotides with and without the CPT treatment, Figure 2b reveals that combining oligonucleotides of Comparative Examples A and B together also did not affect the viability of the cells and the reduction in viability seen with this combination after CPT treatment could be associated to the CPT action alone.

### SiRNA sequences used in the experiments:

**SiRNA sequences: Hs-PPEF1-2: ATCGAATATGCTGATGAACAA (SEQ ID NO 1)**
**Hs-PPEF1-5: CAGTTCGAATCTGGTAAACAT (SEQ ID NO 2)**
**Target gene ID: 5475 (SEQ ID NO 3)**

Meanwhile the combination of oligonucleotides of Examples 1 and 2 led to comparable reduction in viability when transfected separately (about 40%) and when this combination treatment was followed by treatment with CPT, there was an additional reduction in viability amounting to a total of about 85% (Figure 2b).

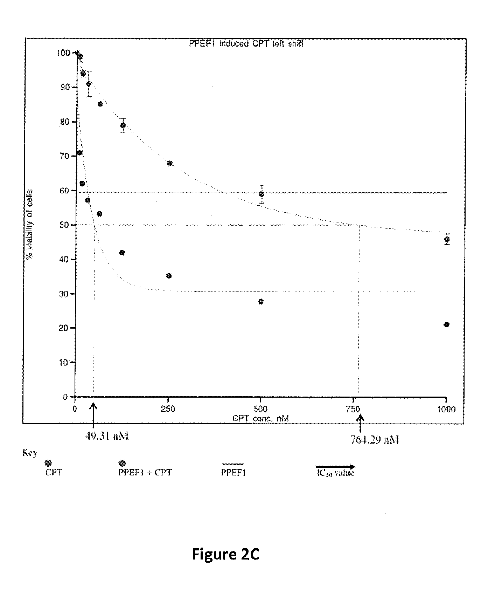

To effectively demonstrate the left shift due to the combination treatment, cells were seeded in 96 well formats and half of the plate was transfected in triplicates with the SiRNA for PPEF1. 24 hrs post transfection the cells both SiRNA transfected and mock transfected were treated with different concentrations (0, 8, 16, 31, 63, 125, 250, 500 and 1000nM) of the drug camptothecin (CPT). 72 hrs post transfection the cells were analyzed for viability and the results (a total of 3 separate repetitions) were plotted using graph-pad prism as indicated in Figure 2c above.

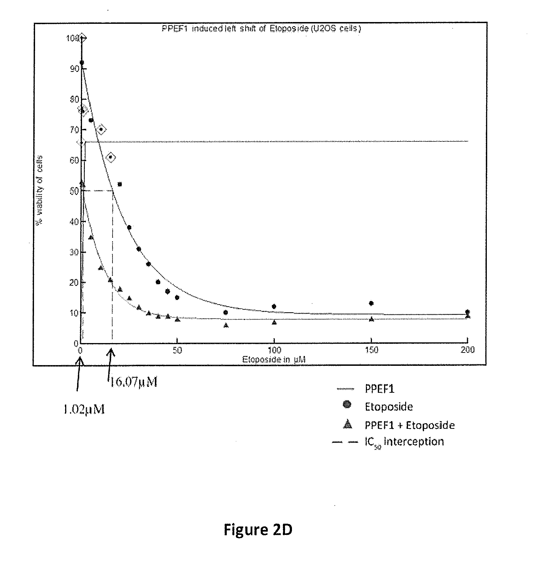

A similar experiment as in 2c was performed using Etoposide (a radiomimetic) as demonstrated above in Figure 2d. It is observed that the SiRNA knockdown sensitizes the cells to Etoposide treatment.

### Confirmation of SiRNA mediated target knockdown

The SiRNA mediated target knockdown efficiency for each candidate phosphatase was analyzed by RT-qPCR method. Briefly, cells were seeded at a density of 5 x 105 cells/7ml growth medium in 10cm dishes. Target phosphatases were depleted by SiRNA method 24hrs post seeding. 72hrs post SiRNA depletion, total RNA was isolated. The RNA was quantified using nanodrop technology and equal amount of total RNA was used to synthesize cDNA. A 1/10 dilution of the cDNA was then used to perform qPCR using target specific primers as well as specific primers for housekeeping genes. The knockdown efficiency was calculated with reference to the control (scrambled SiRNA) treated cells after normalization with the housekeeping genes and expressed as percentage. Only targets with knockdown efficiency of more than 80% were considered worth further screening. The target phosphatase investigated here repeatedly indicated a knockdown efficiency of between 90 - 94%.

### Secondary screening

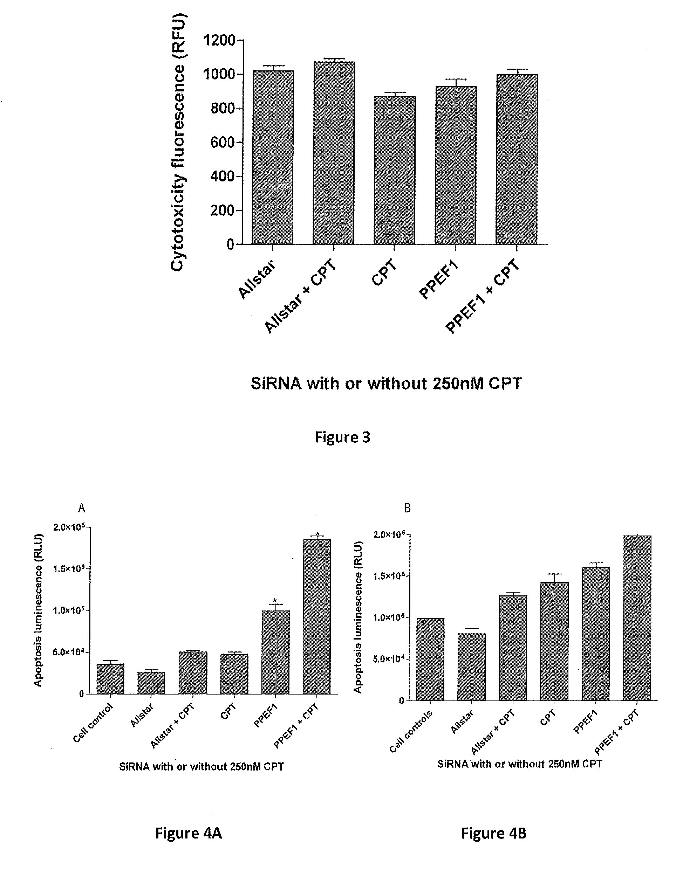

The secondary screening is designed in part to confirm the primary screening and to provide an indication as to what may be happening due to the phosphatase knockdown treatment. We utilized the Promega ApoTox-Glo^{™} triplex assay. In principle this assay combines three Promega assay techniques to assess viability, cytotoxicity and caspase activation events within a single assay well. The first part of this assay simultaneously measures two protease activities wherein one is a marker for viability and the other a marker for cytotoxicity. The live-cell protease activity is limited to intact viable cells and is measured using a cell permeable peptide substrate (glycyl-phenylalanyl-aminofluorocoumarin; GF-AFC) which is fluorogenic. This live cell protease becomes inactive upon loss of cell membrane integrity and leakage into the surrounding cell culture medium. A second, fluorogenic cell-impermeable peptide substrate (bis-alanylalanylphenylalanyl-rhodamine 110; bis-AAF-R110) is used to measure dead cell protease activity, which is released from cells that have lost membrane integrity. Because bis-AFF-R110 is not cell permeable, essentially no signal from this substrate is generated by intact viable cells. The live and dead cell proteases produce different products, AFC and R110 with different excitation and emission spectra, permitting simultaneous detection. The second part of the assay uses the caspase-Glo assay technology by providing a luminogenic caspase 3/7 substrate containing the tetra-peptide sequence DEVD in a reagent optimized for caspase activity, luciferase activity and cell lysis. Adding the caspase-Glo 3/7 reagent in an "add-mix-measure" format results in cell lysis followed by caspase cleavage of the substrate and the generation of a "glow-type" luminescence signal produced by luciferase. Luminescence is proportional to the amount of caspase activity present in each well of the plate. The protocol can be found at http://www.promega.com/resources/protocols/technical-manuals/101/apotox-glo-triplex-assay-protocol/. The SiRNA mediated knockdown of the PPEF1 phosphatase induced a viability reduction of about 40% on its own and when combined with 250nM of CPT the total viability was reduced by 72%. Therefore we examined this effect using the Apotox-glo triple assay reagent described above. Figures 3 and 4 represent data obtained from the treatment of U2OS cells with the SiRNA mediated knockdown of the phosphatase PPEF1 and analyzed with the ApoTox-Glo-triplex assay.

In Figure 3, we evaluated the extent of cytotoxicity caused by various agents used in the treatment of the cells. The result indicated no significant difference in the amount of cytotoxicity between the different treatments applied. Taken together, we conclude this observation to mean that the difference observed in Figure 4a when comparing cells treated with the PPEF1 SiRNA to those treated with CPT (a genotoxic compound) is due to a specific effect of the phosphatase inhibition. Additionally, we observed a significant enhancement of this effect when PPEF1 inhibition was followed by CPT treatment. Furthermore, longer incubation led to increased apoptosis as seen in Figure 4b below.

### Q-PCR profiling

Bolstered by the observations above, we have examined the gene expression profile of cells transfected with the target SiRNA and treated with or without CPT. On this basis a preliminary test using the Qiagen's RT2-qPCR profiler system was performed. This system offers the possibility to assess the activity of 84 genes in one experiment. Our pilot experiment with the RT2-qPCR profiler (data not shown) indicated that the SiRNA mediated knockdown of the phosphatase PPEF1 up regulates some relevant genes such as the gene encoding the death associated protein kinase 1 and TP73 gene - a member of the p53 family of proteins (DAPK1 and TP73) whose involvement in the induction of cancer related apoptosis has been reported.

### Tertiary screening

### A. Phosphorylation of histone H2AX and DNA damage response

Phosphorylation of the histone H2AX (Ser-139) has been earmarked a sign for DNA double strand break (Modesti and Kanaar, 2001; Rogakou et al., 1999). Phosphorylated H2AX protein is called γ-H2AX and it is rapidly formed after cells have been treated with ionizing radiation and cells undergoing apoptosis due to DNA damage (Paull et al., 2000). The current experimental findings demonstrate that the knockdown of the phosphatase PPEF1 alone or in combination with CPT induces apoptosis. It was intended herein to investigate γ- H2AX formation in cells after PPEF1 SiRNA treatment on its own and in combination with CPT. Cells were seeded out, transfected and treated in triplicates according the established protocol.

Initially, a time course was made to monitor the formation of γ-H2AX. The cells were to be analyzed for the formation of γ-H2AX protein by using the following methods; (1) Flow cytometry method, Western blotting method and immunofluorescence staining. In the case of an indication of an increase in the formation of γ-H2AX or its reduction after the treatment, the status of the upstream kinases responsible for its phosphorylation such as ATR, DNA-PK and ATM will be analyzed by Western blot. The PPEF1 phosphatase inhibition will be classified as either enhancing the γ-H2AX formation or not. Additionally, the various checkpoint kinases (Chk1 and Chk2) will equally be examined by Western blot.

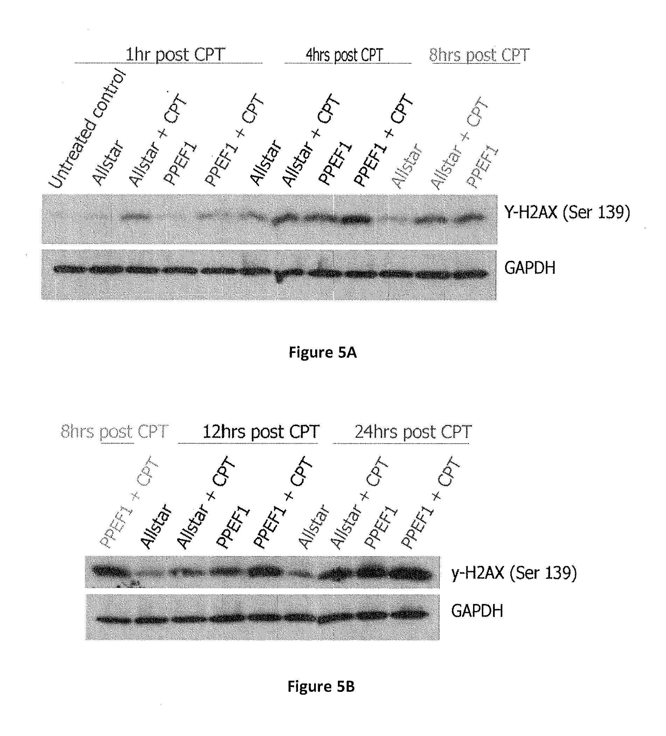

In Figures 5a and 5b we observe that cells that were transfected with the control SiRNA Allstar demonstrated only basal phosphorylation of the histone H2AX when compared across the different time points. Remarkably we see a steady state increase of phosphorylation of the histone H2AX when cells were transfected with the SiRNA for PPEF1 with time as compared to the unspecific SiRNA control (Allstar: a validated non-silencing SiRNA obtained from Qiagen). Additionally, CPT is known to induce DNA double strand break (DSB) and as a consequence induce the phosphorylation of histone H2AX as a response to repair mechanism. We have previously observed that the combination of PPEF1 knockdown with minimum CPT treatment increases cell death by apoptosis. The phosphorylation of H2AX by PPEF1 on its own confirms our observation that cells treated to inhibit the activity of PPEF1, lose viability and die by apoptosis.

### PPEF1 inhibition sensitizes proven drug resistant cancer cell lines

In addition to the described experiments, the effect of inhibiting the phosphatase PPEF1 on characterized published drug resistant cell lines was assessed. The resistant cell lines used in these experiments were DU145/RCO.1 cells, which were kindly provided by Prof.Dr. Yves Pommier from the Laboratory of Molecular Pharmacology, Center of Cancer research, National Cancer Institute, Bethesda, USA. These DU145/RCO.1 cells and their resistance to CPT) have been described Urasaki et al. (Cancer Research 61, 2001, pp. 1964-1969).

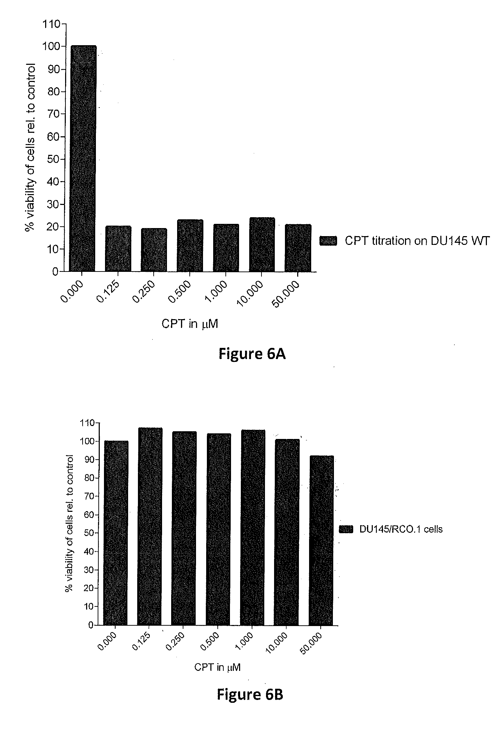

Initially, DU145 prostate cancer wild type cell line was treated with varying concentrations of camptothecin (CPT). In Figure 6a, it is demonstrated that the viability of DU145 cells is greatly reduced. When given the same treatments to DU145/RCO.1 cells resistant to CPT, the viability did not significantly change as can be seen from Figure 6b.

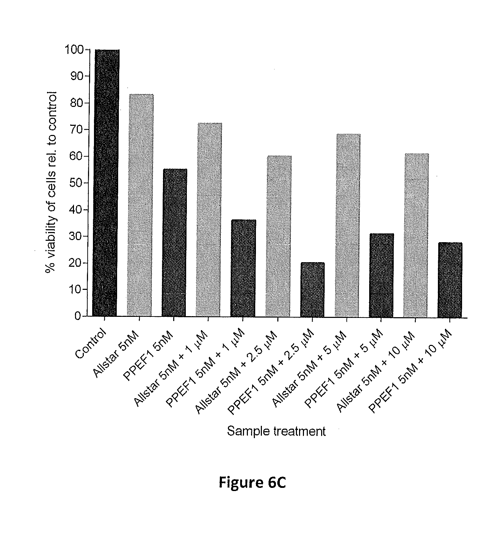

When the phosphatase PPEF1 was inhibited by treatment with PPEF1-5 (Example 2) using the SiRNA method in Camptothecin resistant prostate cancer cell line DU145/RCO.1, it was observed that the cells became sensitive and lost viability from equivalent concentration of CPT which otherwise would be resistant. The results of these experiments can be seen in Figure 6c.

The following sequences are polynucleotides of the invention encoding amino acid sequences comprised in the antigen binding molecules of the invention.

| **Description** | **Nucleotide sequence** | **Seq. ID #** |
|---|---|---|
| SiRNA PPEF1-2 | ATCGAATATGCTGATGAACAA | **1** |
| SiRNA PPEF1-5 | CAGTTCGAATCTGGTAAACAT | **2** |
| PPEF1 gene sequence | ATGGGATGCAGCAGTTCTTCAACGAAAACCAGGAGATCTGACACATCACT GAGAGCTGCGTTGATCATCCAGAACTGGTACCGAGGTTACAAAGCTCGAC TGAAGGCCAGACAACACTATGCCCTCACCATCTTCCAGTCCATCGAATAT GCTGATGAACAAGGCCAAATGCAGTTATCCACCTTCTTTTCCTTCATGTT GGAAAACTACACACATATACATAAGGAAGAGCTAGAATTAAGAAATCAGT CTCTTGAAAGCGAACAGGACATGAGGGATAGATGGATTATGTGGACTCG ATAGATGTCCCAGACTCCTATAATGGTCCTCGGCTACAATTTCCTCTCAC TTGTACGGATATTGATTTACTTCTTGAGGCCTTCAAGGAACAACAGATAC TTCATGCCCATTATGTCTTAGAGGTGCTATTTGAAACCAAGAAAGTCCTG AAGCAAATGCCGAATTTCACTCACATACAAACTTCTCCCTCCAAAGAGGT AACAATCTGTGGTGATTTGCATGGAAACTGGATGATCTTTTTTTGATCT TCTACAAGAATGGTCTCCCCTCAGAGAGGAACCCGTATGTTTTTAATGGT GACTTTGTAGATCGAGGAAAGAATTCCATAGAGATCCTAATGATCCTGTG TGTGAGTTTTCTTGTCTACCCCAATGACCTGCACTTGAACAGAGGGAACC ACGAAGATTTTATGATGAATCTGAGGTATGGCTTCACGAAAGAAATTTTG CATAAATATAAGCTACATGGAAAAAGAATCTTACAAATCTTGGAAGAATT CTATGCCTGGCTCCCAATCGGTACAATCGTTGACAATGAAATCCTGGTCA TCCATGGTGGGATATCAGAGACCACAGACTTGAATTTACTCCACCGTGTA GAGAGGAACAAGATGAAATCTGTGCTGATACCACCAACGGAAACAAACAG AGACCATGACACTGACTCGAAGCACAATAAAGTAGGTGTGACTTTTAATG CACATGGAAGAATCAAAACAAATGGATCTCCTACTGAACACTTAACAGAG CATGAATGGGAACAGATTATTGATATTCTGTGGAGTGATCCCAGAGGCAA AAATGGCTGTTTTCCAAATACGTGCCGAGGAGGGGGCTGCTATTTTGGAC CAGATGTTACTTCCAAGATTCTTAATAAATACCAGTTGAAGATGCTCATC AGGTCTCATGAATGTAAGCCCGAAGGGTATGAAATCTGTCATGATGGGAA GGTGGTGACTATATTTTCTGCTTCTAATTATTATGAAGAAGGCAGCAATC GAGGAGCTTACATCAAACTATGTTCTGGTACAACTCCTCGATTTTTCCAG TACCAAGTAACTAAAGCAACGTGCTTTCAGCCTCTTCGCCAAAGAGTGGA TACTATGGAAAACAGCGCCATCAAGATATTAAGAGAGAGAGTGATTTCAC GAAAAAGTGACCTTACTCGTGCTTTCCAACTTCAAGACCACAGAAAATCA GGAAAACTTTCTGTGAGCCAGTGGGCTTTTTGCATGGAGAACATTTTGGG GCTGAACTTACCATGGAGATCCCTCAGTTCGAATCTGGTAAACATAGACC AAAATGGAAACGTTGAATACATGTCCAGCTTCCAGAATATCCGCATTGAA AAACCTGTACAAGAGGCTCATTCTACTCTAGTTGAAACTCTGTACAGATA CAGATCTGACCTGGAAATCATATTTAATGCCATTGACACTGATCACTCAG GCCTGATCTCCGTGGAAGAATTTCGTGCCATGTGGAAACTTTTTAGTTCT CACTACAATGTTCACATTGATGATTCCCAAGTCAATAAGCTTGCCAACAT AATGGACTTGAACAAAGATGGAAGCATTGACTTTAATGAGTTTTTAAAGG CTTTCTATGTAGTGCATAGATATGAAGACTTGATGAAACCTGATGTCACC AACCTTGGCTAA | **3** |
| PPEF1 RNA sequence | AUGGGAUGCAGCAGUUCUUCAACGAAAACCAGGAGAUCUGACACAUCACU GAGAGCUGCGUUGAUCAUCCAGAACUGGUACCGAGGUUACAAAGCUCGAC UGAAGGCCAGACAACACUAUGCCCUCACCAUCUUCCAGUCCAUCGAAUAU GCUGAUGAACAAGGCCAAAUGCAGUUAUCCACCUUCUUUUCCUUCAUGUU GGAAAACUACACACAUAUACAUAAGGAAGAGCUAGAAUUAAGAAAUCAGU CUCUUGAAAGCGAACAGGACAUGAGGGAUAGAUGGGAUUAUGUGGACUCG AUAGAUGUCCCAGACUCCUAUAAUGGUCCUCGGCUACAAUUUCCUCUCAC | **4** |
| | UUGUACGGAUAUUGAUUUACUUCUUGAGGCCUUCAAGGAACAACAGAUAC UUCAUGCCCAUUAUGUCUUAGAGGUGCUAUUUGAAACCAAGAAAGUCCUG AAGCAAAUGCCGAAUUUCACUCACAUACAAACUUCUCCCUCCAAAGAGGU AACAAUCUGUGGUGAUUUGCAUGGGAAACUGGAUGAUCUUUUUUGAUCU UCUACAAGAAUGGUCUCCCCUCAGAGAGGAACCCGUAUGUUUUUAAUGGU GACUUUGUAGAUCGAGGAAAGAAUUCCAUAGAGAUCCUAAUGAUCCUGUG UGUGAGUUUUCUUGUCUACCCCAAUGACCUGCACUUGAACAGAGGGAACC ACGAAGAUUUUAUGAUGAAUCUGAGGUAUGGCUUCACGAAAGAAAUUUUG CAUAAAUAUAAGCUACAUGGAAAAAGAAUCUUACAAAUCUUGGAAGAAUU CUAUGCCUGGCUCCCAAUCGGUACAAUCGUUGACAAUGAAAUCCUGGUCA UCCAUGGUGGGAUAUCAGAGACCACAGACUUGAAUUUACUCCACCGUGUA GAGAGGAACAAGAUGAAAUCUGUGCUGAUACCACCAACGGAAACAAACAG AGACCAUGACACUGACUCGAAGCACAAUAAAGUAGGUGUGACUUUUAAUG CACAUGGAAGAAUCAAAACAAAUGGAUCUCCUACUGAACACUUAACAGAG CAUGAAUGGGAACAGAUUAUUGAUAUUCUGUGGAGUGAUCCCAGAGGCAA AAAUGGCUGUUUUCCAAAUACGUGCCGAGGAGGGGGCUGCUAUUUUGGAC CAGAUGUUACUUCCAAGAUUCUUAAUAAAUACCAGUUGAAGAUGCUCAUC AGGUCUCAUGAAUGUAAGCCCGAAGGGUAUGAAAUCUGUCAUGAUGGGAA GGUGGUGACUAUAUUUUCUGCUUCUAAUUAUUAUGAAGAAGGCAGCAAUC GAGGAGCUUACAUCAAACUAUGUUCUGGUACAACUCCUCGAUUUUUCCAG UACCAAGUAACUAAAGCAACGUGCUUUCAGCCUCUUCGCCAAAGAGUGGA UACUAUGGAAAACAGCGCCAUCAAGAUAUUAAGAGAGAGAGUGAUUUCAC GAAAAAGUGACCUUACUCGUGCUUUCCAACUUCAAGACCACAGAAAAUCA GGAAAACUUUCUGUGAGCCAGUGGGCUUUUUGCAUGGAGAACAUUUUGGG GCUGAACUUACCAUGGAGAUCCCUCAGUUCGAAUCUGGUAAACAUAGACC AAAAUGGAAACGUUGAAUACAUGUCCAGCUUCCAGAAUAUCCGCAUUGAA AAACCUGUACAAGAGGCUCAUUCUACUCUAGUUGAAACUCUGUACAGAUA CAGAUCUGACCUGGAAAUCAUAUUUAAUGCCAUUGACACUGAUCACUCAG GCCUGAUCUCCGUGGAAGAAUUUCGUGCCAUGUGGAAACUUUUUAGUUCU CACUACAAUGUUCACAUUGAUGAUUCCCAAGUCAAUAAGCUUGCCAACAU AAUGGACUUGAACAAAGAUGGAAGCAUUGACUUUAAUGAGUUUUUAAAGG CUUUCUAUGUAGUGCAUAGAUAUGAAGACUUGAUGAAACCUGAUGUCACC AACCUUGGCUAA | |

## Claims

1. A combination of a PPEF inhibitor capable of causing apoptosis in cancer cells and an active pharmaceutical ingredient capable of causing apoptosis in cancer cells.

2. The combination according to claim 1 wherein the IC50 value of the combination is at least 10 times lower than the IC50 value of the active pharmaceutical ingredient alone.

3. The combination according to any one of claims 1 and 2 wherein the PPEF inhibitor is a PPEF1 inhibitor.

4. The combination according to any one of the preceding claims, wherein the PPEF inhibitor is selected from an oligonucleotide selected from an antisense oligonuncleotide, a SiRNA oligonucleotide and a triplex forming oligonucleotide.

5. The combination according to any one of the preceding claims, wherein the PPEF inhibitor is a SiRNA oligonucleotide.

6. The combination according to claim 5, wherein the SiRNA,oligonucleotide is selected from a group consisting of SEQ ID NO 1 and SEQ ID NO 2.

7. The combination according to any one of the preceding claims, wherein the active pharmaceutical ingredient is selected from a DNA damaging agents and a topoisomerase inhibitor.

8. The combination according to any one of the preceding claims, wherein the active pharmaceutical ingredient is selected from cisplatin, camptothecin and etoposide.

9. A pharmaceutical composition comprising the combination of any one of the preceding claims, and a pharmaceutically acceptable carrier.

10. Use of the combination according to any one of claims 1 to 8 or the composition of claims 9 as a medicament.

11. Use of the combination according to any one of claims 1 to 8 or the composition of claims 9 in the treatment of cancer.

12. Use of a PPEF inhibitor in the treatment of cancer, the PPEF inhibitor being capable of reducing the viability of cancer cells by at least 10% compared to a treatment without the PPEF inhibitor.

13. Use of a PPEF inhibitor to sensitize cancer cells which are resistant to an active pharmaceutical ingredient capable of causing apoptosis in non-resistant cancer cells.

14. PPEF inhibitor for use as a medicament, the PPEF inhibitor being capable of reducing the viability of cancer cells by at least 10% compared to a treatment without the PPEF inhibitor.

15. PPEF inhibitor according to claim 14 wherein the PPEF inhibitor is a SiRNA oligonucleotide.
